# EUROPEAN PATENT SPECIFICATION

(11) **EP 1 278 891 B1**
(45) Date of publication and mention of the grant of the patent: **16.09.2009**
(21) Application number: 01933906.8
(22) Date of filing: 30.04.2001
(51) Int. Cl.: C12Q 1/68, G01N 33/68

(54) **METHOD AND PROBES FOR THE GENETIC DIAGNOSIS OF HEMOCHROMATOSIS**
VERFAHREN UND SONDEN ZUR DIAGNOSE VON HÄMOCHROMATOSE
PROCEDE ET SONDES DESTINES AU DIAGNOSTIC GENETIQUE DE L'HEMOCHROMATOSE

(30) Priority: 02.05.2000 AT 7662000; 08.05.2000 AT 7992000
(43) Date of publication of application: 29.01.2003
(60) Divisional application: 09165173.7
(73) Proprietor: ViennaLab Diagnostics GmbH, 1120 Vienna (AT)
(72) Inventor: PIPERNO, Alberto, I-20124 Milano (IT); GASPARINI, Paolo, Brandico (IT); CAMASCHELLA, Clara, I-10128 Torino (IT); DE VILLIERS, Nico, 7580 Western Cape Province (ZA); OBERKANINS, Christian, A-1060 Wien (AT); KURY, Friedrich, A-1180 Wien (AT)
(74) Representative: Redl, Gerda
(86) International application number: PCT/EP2001/004835
(87) International publication number: WO 2001/083812

(56) References cited:
- WO-A-97/38137
- WO-A-98/14466
- DE VILLIERS J. ET AL: "Spectrum of mutations in the HFE gene implicated in heamochromatosis and porphyria" HUMAN MOLECULAR GENETICS, vol. 8, no. 8, August 1999 (1999-08), pages 1517-22, XP002175621 cited in the application
- SANCHEZ ET AL: "PREVALENCE OF THE Cys282Tyr AND His63Asp HFE GENE MUTATIONS IN SPANISH PATIENTS WITH HEREDITARY HEMOCHROMATOSIS" JOURNAL OF HEPATOLOGY, XX, XX, vol. 29, no. 5, November 1998 (1998-11), pages 725-728, XP002113587
- MURA C ET AL: "HFE mutations analysis in 711 hemochromatosis probands: evidence for S65C implication in mild form of hemochromatosis" BLOOD, W.B.SAUNDERS COMPAGNY, ORLANDO, FL, US, vol. 93, no. 8, 15 April 1999 (1999-04-15), pages 2502-2505, XP002143645 ISSN: 0006-4971 cited in the application
- BARTON J C ET AL: "TWO NOVEL MISSENSE MUTATIONS OF THE HFE GENE (I05T AND G93R) AND IDENTIFICATION OF THE S65C MUTATION IN ALABAMA HEMOCHROMATOSIS PROBANDS" BLOOD CELLS, MOLECULES AND DISEASES, LAJOLLA, US, vol. 25, no. 9, 1999, pages 147-155, XP000929556 ISSN: 1079-9796 cited in the application
- RUBINI M ET AL: "A novel mutation of HFE explains the phenotype of hereditary hemochromatosis in a C282Y carrier" EUROPEAN JOURNAL OF HUMAN GENETICS, vol. 8, no. Suppl 1, 2000, page 158 XP001016153 European Human Genetics Conference 2000 Amsterdam May 27-Feb. 30 cited in the application
- CAMASCHELLA C ET AL: "The gene TFR2 is mutated in a new type of haemochromatosis mapping to 7q22" NATURE GENETICS, vol. 25, - 1 May 2000 (2000-05-01) pages 14-15, XP002185806
- FLEMING R ET AL: "Transferrin receptor 2: continued expression in mouse liver in the face of iron overload and in hereditary hemochromatosis" PROCEEDINGS OF THE NATIONAL ACADEMY OF SCIENCES, USA, vol. 97, no. 5, February 2000 (2000-02), pages 2214-19, XP002185807

## Description

The invention relates to a method for the genetic diagnosis of hemochromatosis as well as to probes for the genetic diagnosis of hemochromatosis.

Hereditary Hemochromatosis (HH) /11/ is a common autosomal recessive disease characterized by a disorder of the iron metabolism in the human body leading to progressive iron overload. The excess iron is deposited in various organs, primarily liver, heart and pancreas, causing irreversible damages and diseases as liver cirrhosis, diabetes, arthritis, cardiomyopathies and premature death. With early diagnosis and treatment by therapeutic phlebotomy, organ damage can be completely prevented and life expectancy of patients is normal. As clinical symptoms of hemochromatosis often appear not till the age of 40, 50 or older, when irreversible damages of organs already may exist, a presymptomatic diagnosis is favourized.

A number of point mutations within a novel MHC class I-like gene, termed HFE (locus 6p), have been identified and related to HH /1,7,9/. This gene encodes a protein that interacts with transferrin receptor (TFRC) and negatively affects cellular iron uptake from transferrin /36/. In 1996, the HFE gene was identified and two mutations have been originally described, C282Y and H63D /1/. These two mutations are presently used for the genetic diagnosis of hemochromatosis.

H63D is a C→ G mutation in codon 63, converting the amino acid 63 from histidine into aspartic acid. Because of its high frequency in the general population, its role in the diagnosis of the disease remains uncertain.

A much better criterion for the diagnosis of hemochromatosis is the C282Y mutation, found in 65 to 95% of the concerned patients. C282Y is a G→ A mutation at nucleotide 845 of the HFE cDNA sequence that converts the amino acid 282 from cysteine into tyrosine. So most HH patients are homozygous for C282Y /1,13,14,15/. Among HH probands, compound heterozygotes C282Y/H63D account for about 5-7% /1,16/, H63D homozygotes are very rare and both have generally a mild form of hemochromatosis /17/. A minority of cases presenting with a HH phenotype are C282Y or H63D heterozygotes or neither C282Y or H63D. This appears to be more common in southern than in northern Europe /18,19/. That means the percentage of hemochromatosis patients not showing a C282Y mutation varies and is e.g. higher in Italy than in countries of Northern Europe. In a collaborative study including 188 Italian patients with HH-like phenotype, 28% were incompletely characterised with at least one chromosome without an assigned mutation /19/. In some of these patients the presence of other HFE mutations or other 6p-linked genetic determinants or the existence of other form of genetic iron overload unlinked to chromosome 6 have been suggested /19,20,21/. Three novel HFE mutations have been recently discovered in the heterozygous state in association with C282Y in single cases with classical HH: a splice site mutation (IVS3 + 1G→T) causing obligate skipping of exon 3 /22/, and two missense mutations (1105T and G93R) located in α1 domain that may affect the binding of the HFE protein to the transferrin receptor /23/. Moreover, the S65C missense mutation was found to be significantly enriched in HH chromosomes that were neither C282Y or H63D, suggesting that the S65C could be another variant contributing to a mild form of HH in association with C282Y or H63D /9/. Other missense mutations have been recently described but their role in the pathogenesis of HH is still unclear /7/.

While homozygozity for C282Y is observed in the majority of Caucasian HH patients and is well recognized as a cause for the disease, the diagnostic value of other HFE mutations is still under investigation. It should be of particular interest to define disease-causing mutations in those HH patients who are not homozygous for C282Y.

It therefore is important to find - apart from the known mutations - those further genetic causes in the HFE gene or other genes, which may lead to the excess of iron in the patients.

For that reason the problem of the present invention is to find further possibilities to diagnose genetic causes for hemochromatosis.

The problem is solved by examining a biological sample for the presence of a G→T mutation at nucleotide 502 of the HFE cDNA sequence. Such mutation has been found during examinations of five unrelated Italian patients heterozygous for C282Y with the classical hemochromatosis phenotype. The mutation (nt 502 G→T or E168X) was identified in exon 3 of the C282Y negative chromosome. The G→ T mutation at nucleotide 502 causes the conversion of the corresponding amino acid 168 from glutamic acid into a stop codon (E168X).

The present disclosure also relates to examining a biological sample for the presence of a G→ C mutation at nucleotide 502 of the HFE cDNA sequence. This exon 3 mutation (nt 502 G→C or E168Q) had been identified in a 79-year old Caucasian female from South-Africa. She was also heterozygous for the H63D mutation and her serum ferritin was moderately elevated at the time of testing (242 ng/ml; ref. values: 12-119 ng/ml). She presented without noteworthy clinical symptoms, but had a family history of both heart disease and different types of cancer. The E168Q mutation was initially identified by single-strand conformation polymorphism (SSCP) analysis /7/ and subsequently confirmed by DNA sequencing. The G→C mutation at nucleotide 502 causes the conversion of the corresponding amino acid 168 from glutamic acid into glutamine (E168Q).

Preferably the biological sample is additionally examined for the presence of a of a G→ A mutation at nucleotide 845 and/or a C→G mutation at nucleotide 187 of the HFE cDNA sequence, so that a diagnosis of hemochromatosis may take place even if only one of these known mutations is found.

The problem is further solved by examining a biological sample for the presence of nucleic acids coding for HFE products with a substitution of glutamic acid with glutamine or a stop codon at amino acid 168 (E168Q, E168X) or examining the biological sample for the presence of a HFE gene product with a substitution of glutamic acid with glutamine or a stop codon at amino acid 168 (E168Q, E168X).

The point mutations known from the state of art or according to the present invention and described above cause certain amino acid substitutions and have been found with patients affected by hemochromatosis. The amino acid substitutions described above however may be created also by mutations different from the said known mutations or mutations according to the present invention and will never the less lead to an affection or a carrier status. Examining a biological sample for the presence of one or more of the above amino acid substitutions, will therefore give information whether the patient the biological sample originates from is affected by or is a carrier of hemochromatosis.

For the biological sample blood, biopsic tissue, smear from the oral cavity, amniotic fluid or the like may be used and the biological sample is subjected to known pretreatments according to the selected known examination procedure.

If the biological sample is also examined for the presence of nucleic acids not showing the mentioned mutations or amino acid substitutions in their HFE gene or respective gene products, it may be proved, whether the mutations are heterozygous or homozygous.

The examination may be accomplished in a known manner by sequence analysis of the nucleic acids derived from the biological sample.

Or nucleic acids of the biological sample are brought into contact with at least one probe capable of hybridizing with a region of said nucleic acids corresponding to a region of the HFE cDNA sequence containing nucleotide 502 if a G→T mutation exists at position 502 of the HFE cDNA sequence and/or containing nucleotide 502 if a G→C mutation exists at position 502 of the HFE gene and an examination is accomplished whether respective hybridization products have been created. From the biological sample nucleic acids (genomic DNA and/or RNA, seperately or as a mixture) are may be made accessible using any of the respective methods known from the state of art.

If the biological sample is to be examined for the presence of the known C282Y and/or H63D mutation too, nucleic acids of the biological sample are also contacted with at least one further probe capable of hybridising with a region of said nucleic acids corresponding to a region of the HFE cDNA sequence containing nucleotide 845 if a G→ A mutation exists at nucleotide 845 and/or nucleotide 187 if a C→G mutation exists at nucleotide 187 of the HFE cDNA sequence and an examination is accomplished whether respective hybridization products have been created.

In order to differentiate whether one or more mutations are heterozygous or homozygous, the nucleic acids of the sample are preferably brought also into contact with at least one further probe capable of hybridising with a region of said nucleic acids corresponding to a region of the HFE cDNA sequence containing at least one of said nucleotides if no mutation exists and an examination is accomplished whether respective hybridization products have been created.

A number of different methods are currently used for the analysis of mutations, among them restriction fragment length polymorphism (RFLP), PCR with sequence-specific primers (PCR-SSP), allele-specific oligonucleotide hybridization, single-strand conformation polymorphism (SSCP), denaturing gradient gel electrophoresis (DGGE), oligonucleotide ligation assay (OLA), real-time fluorescence PCR, and DNA sequencing /1,2,3,4,5,6,7,8,9/. If RNA is examined, this may be accomplished for example by "NASBA" or RNA is transcribed into cDNA by reverse transcripton, which is amplified thereafter by PCR and which is characterized successively. All these known analysis methods may be used for the method for diagnosis of the present invention.

The probe for the diagnosis of hemochromatosis by means of mutations in the HFE gene according to the invention is capable of hybridising with nucleic adds of a biological sample in a region corresponding to a region of the HFE cDNA sequence containing nucleotide 502 to determine if a G→T mutation exists at position 502 of the HFE cDNA sequence.

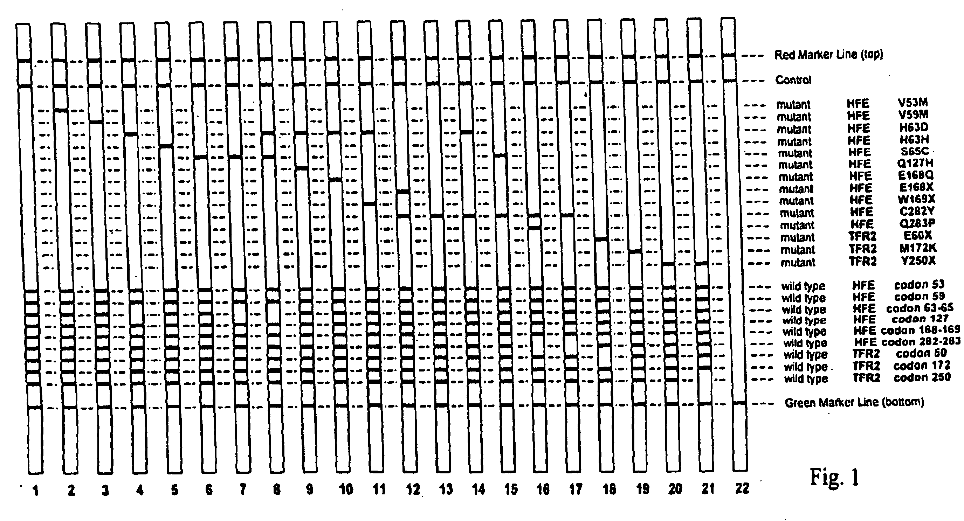

The following examples describe performance possibilities for the methods for the diagnosis of hemochromatosis according to the invention. The enclosed drawing helps to illustrate the examples. The graphic illustration of Fig. 1 shows some possible staining patterns obtainable with DNAs carrying different mutant and wild-type HFE alleles using reverse hybridization as explained in example 2.

### EXAMPLE 1:

### DNA Isolation and Amplification:

DNA was isolated from anticoagulated blood using standard extraction methods /10/ or commercially available reagents (GenXtract DNA extraction system, ViennaLab). HFE gene exon 3 sequences were amplified in a PCR reaction using primers 5'-GGA TTG GAG AGC AGC AGA AC-3' and 5'-AAA CTC CAA CCA GGG ATT CC-3'. Each primer was used at a final concentration of 0.4µM in 1xPCR buffer containing 1.5mM Mg2+, 200 µM dNTPs (Epicentre, Madison, WI) and 0.02 U/µl AmpliTaq DNA polymerase (PE Biosystems, Foster City, CA). A thermocycling program of 30 cycles (94°C for 15s, 58°C for 30s, and 72°C for 30s) was performed on the GeneAmp PCR System 2400 (PE Biosystems).

### Sequencing:

PCR products were purified with "Centricon-100" columns according to the manufacturers recommendations. Amplified and purified DNA was quantitated by UV-spectrophotometry and subsequently sequenced strictly following the protocol described in the "BigDye Terminator Cycle Sequencing Ready Reaction Kit - with AmpliTaq DNA Polymerase, FS" (PE Applied Biosystems) for the ABl Prism instrumentation (PE Applied Biosystems).

### EXAMPLE 2:

Hereafter it is reported about the successful application of reverse hybridization, to set up a screening assay for the detection of some known mutations and some new HFE and TFR2 point mutations as well as a new TFR2 frameshift mutation. The test is based on multiplex DNA amplification and ready-to-use membrane teststrips, which contain oligonucleotide probes for each wild-type and mutated allele immobilized as an array of parallel lines. The test is rapid, easy to perform and accessible to automation on commercially available equipment, and by adding new probes the test-strip can easily be adapted to cover an increasing number of mutations.

### DNA Isolation and Amplification:

DNA was isolated from anticoagulated blood using standard extraction methods /10/ or commercially available reagents (GenXtract DNA extraction system, ViennaLab). HFE gene exon 2, 3 and 4 sequences and TFR2 gene exon 2, 4 and 6 sequences were amplified in a single, multiplex PCR reaction using 5'-biotinylated primers (/1,31,32/; table 3). Each primer was used at a final concentration of 0.4 µM in 1x PCR buffer including 1.5 mM Mg2+, 200 µM dNTPs (Epicentre, Madison Wl), and 0.02 U/µl AmpliTaq DNA polymerase (PE Biosystems, Foster City, CA). A thermocycling program of 30 cycles (94°C for 15 s, 58°C for 30 s and 72°C for 30 s) was performed on the GeneAmp PCR System 2400 (PE Biosystems).

### Preparation of Teststrips:

The general principle of tailing and immobilizing sequence-specific hybridization probes was described previously /34/. Probes (16-24 mers) specific for the wild-type or mutant allele of each mutation were selected from the HFE and TFR2 databank sequences (GenBank accession numbers: HFE-Z92910, TFR2-AF067864) and synthesized using standard phosphoramidite chemistry. A 3'-poly(dT) tail (300-500 residues) was enzymatically added by incubating oligonucleotides (200 pmol) in the presence of 60 U terminal deoxynucleotidyl transferase (Amersham Pharmacia, Buckinghamshire, UK) and 150 nmol dTTP (Epicentre) for 8 h at 37°C. The reaction was terminated by adding EDTA to 10 mM, and tailing efficiency was monitored by agarose gel electrophoresis. Poly(dT)-tailed probes were applied onto nitrocellulose membrane sheets (Whatman, Maidstone, UK) as individual parallel lines of 1 mm width using a custom-made slot-blot apparatus, dried and fixed by overnight baking at 60°C. A 5'-biotinylated control oligonucleotide was included to allow performance control of the detection reagents. The membrane sheet was finally sliced into 3 mm ready-to-use teststrips.

### Reverse-Hybridization and Detection:

The entire hybridization and detection procedure was carried out in individual lanes of a thin-walled plastic incubation tray (Bio-Rad, Hercules, CA). Equal volumes (10 µl) of PCR product and denaturing solution (400 mM NaOH, 10 mM EDTA) were mixed and incubated at room temperature for 5 min. The alkaline reaction was neutralized with sodium phosphate buffer, pH 7, a teststrip was added and hybridization was carried out for 30 min. at 45°C in a shaking waterbath. After three stringent washes at 45°C, bound PCR fragments were detected using a streptavidin-alkaline phosphatase conjugate and color substrates (NBT/BClP). Upon positive reaction, a purple staining was visible after 15 min. incubation at room temperature.

### Results:

A multiplex PCR system was established to obtain biotinylated DNA fragments spanning exons 2, 3 and 4 of the HFE gene and exons 2, 4 and 6 of the TFR2 gene in a single amplification reaction. To avoid potential mistyping of the C282Y mutation due to an intronic polymorphism (5569G→ A) within the binding region of the original reverse primer of Feder et al. (1996)/1/ the exon 4 fragment was modified according to Jeffrey et al. (1999)/32/.

To develop the reverse-hybridization assay DNA samples of individuals known to carry the following exonic HFE mutations were collected: HFE: V53M, V59M, H63D, H63H, S65C, Q127H, E168Q, E168X, W169X, C282Y, Q283P; TFR2: E60X, M172K and Y250X (Table 1).

**Table 1**

| Line | Probe specificity | Mutation designation | Reference |
|---|---|---|---|
| | (control) | | |
| 1 | nt 157 G→ A exon 2 | HFE V53M | Villiers et al. /7/ |
| 2 | nt 175 G→ A exon 2 | HFE V59M | Villiers et al. /7/ |
| 3 | nt 187C→ G exon 2 | HFE H63D | Feder et al./1/ |
| 4 | nt 189 T→ C exon 2 | HFE H63H | Villiers et al. /7/ |
| 5 | nt 193 A→ T exon 2 | HFE S65C | Mura et al. /9/ |
| 6 | nt 381 A→ C exon 3 | HFE Q127H | Villiers et al. /7/ |
| 7 | nt 502 G→C exon 3 | HFE E168Q | present invention |
| 8 | nt 502 G→ T exon 3 | HFE E168X | present invention |
| 9 | nt 506 G→ A exon 3 | HFE W169X | new |
| 10 | nt 845 G→ A exon 4 | HFE C282Y | Feder et al./1/ |
| 11 | nt 848 A→ C exon 4 | HFE Q283P | Rubini et al. /41/ |
| 12 | nt 84-88insC ex 2 | TFR2 E60X | new |
| 13 | nt 515 T A exon 4 | TFR2 M172K | new |
| 14 | nt 750 C G exon 6 | TFR2 Y250X | new |
| 15 | HFE wild-type codon 53 | | |
| 16 | HFE wild-type codon 59 | | |
| 17 | HFE wild-type codon 63-65 | | |
| 18 | HFE wild-type codon 168-169 | | |
| 19 | HFE wild-type codon 282-283 | | |
| 20 | TFR2 wild-type codon 60 | | |
| 21 | TFR2 wild-type codon 172 | | |
| 22 | TFR2 wild-type codon 250 | | |

A variety of candidate probes between 16 to 24 nucleotides in length encoding either the wild-type or mutant allele of each mutation were chosen from the GenBank sequences for HFE and TFR2. After enzymatic poly(dT)-tailing the oligonucleotides were applied to a nitrocellulose membrane as an array of parallel lines and fixed by baking. Finally, the membrane sheet was sliced into individual teststrips. Amplification products from different DNA samples covering all 14 mutations were hybridized to these preliminary teststrips under identical and accurately controlled stringency conditions. Bound biotinylated sequences were detected using a streptavidin-enzyme conjugate and color reaction. From these results, the final teststrip was designed by selecting probes which specifically recognized either the wild-type or the mutant allele under the same standardized conditions. A control probe containing biotin residues was included to react positive irrespective of the presence of hybridizing DNA fragments to control the performance of the detection system. The graphic illustration of Fig. 1 shows some possible staining patterns obtainable with DNAs carrying different mutant and wild-type HFE or TFR2 alleles.

The following samples were used:
1. non-mutant
2. HFE:V53M heterozygous
3. HFE:V59M heterozygous
4. HFE:H63D homozygous
5. HFE:H63H heterozygous
6. HFE:S65C heterozygous
7. HFE:S65C homozygous
8. HFE:H63D + HFE:S65C compound heterozygous
9. HFE:H63D + HFE:Q127H compound heterozygous
10. HFE:H63D + HFE:E168Q compound heterozygous
11. HFE:H63D + HFE:W169X compound heterozygous
12. HFE:E168X + HFE:C282Y compound heterozygous
13. HFE:C282Y heterozygous
14. HFE:H63D + HFE:C282Y compound heterozygous
15. HFE:S65C + HFE:C282Y compound heterozygous
16. HFE:C282Y + HFE:Q283P compound heterozygous
17. HFE:C282Y homozygous
18. TFR2:E60X homozygous
19. TFR2: M172K heterozygous
20. TFR2:Y250X heterozygous
21. TFR2:Y250X homozygous
22. negative PCR control

Most of the known techniques are very limited in their capacity to identify more than one mutation in a single run, therefore a comprehensive genetic analysis may become rather labour intensive. On the other hand, highly informative methods such as DNA sequencing are not suitable for routine diagnostic laboratories where high sample throughput is required. With the above described assay the reverse-hybridization technique was successfully applied to analyze 14 different mutations (11 HFE mutations, 3 TFR2 mutations) simultaneously in a single experiment. In the past this method has been extensively used for genotyping of complex hereditary diseases, such as cystic fibrosis /30/ and thalassemia /33/, as well as infectious agents, such as hepatitis C virus /35/.

The staining pattern of a mutant and its corresponding wild-type probe allows the discrimination of three possible genotypes: the mutation is absent if only the wild-type probe stains positive, both signals are visible in heterozygotes, and only the mutant probe is positive in homozygous mutant samples. The same principle applies to the majority of compound heterozygotes for two mutations (Fig. 1, lanes 8-12, 14-16). However, the HFE gene contains at least two regions, one in exon 2 (nt 187-193) and the other in exon 3 (nt 502-506), where several mutations have been found in close proximity. As the hybridization probes span more than one mutation in this area, no wild-type signal is observed if two mutations from the same cluster (e.g. H63D and S65C) occur on different chromosomes in the same sample (Fig. 1, lane 8). The occurance of two neighbouring mutations on a single chromosome would again lead to a different pattern, but has not been described to date.

The reverse-hybridization assay presented here has the potential of becoming a valuable tool for routine diagnosis of multiple HFE and/or TFR2 mutations. By utilizing a single, multiplex amplification reaction and premade, ready-to-use teststrips the procedure is simple, reliable, and can be accomplished within a few hours from blood drawing to final results. For high sample througput, testing can be automated on existing, commercially available equipment, such as the profiBlot II T (TECAN AG, Hombrechtikon, Switzerland). The number of mutations covered by the present assay can easily be extended using the same approach to select additional hybridization probes.

### EXAMPLE 3:

Five Italian probands affected by HH who were heterozygous for C282Y and negative for H63D were studied. They all met the classical phenotypic criteria for homozygous HH:
1)increased transferrin saturation (repeatedly >50% at fast) and increased serum ferritin levels;
2)hepatocellular hemosiderin deposits of grade III or IV according to Scheuer et al. /24/;
3)hepatic iron index ≥2.

Iron loading anemias, B and C chronic viral hepatitis and a history of heavy alcohol intake or of blood transfusions were excluded. All patients and relatives gave their informed consent to the study.

In clinical studies the presence of fibrosis and cirrhosis was determined at liver biopsy. All cirrhotics were in Child's A class. The presence of clinical complications related to HH was determined according to previously described criteria /25/. Transferrin saturation and serum ferritin were measured by standard methods. Hepatic iron concentration (HIC) was determined by atomic absorption spectrophotometry (Perkin-Elmer S2380, Norwak, CT) and the hepatic iron index (HII) (ratio of HIC [µmol/g dry wt] and age [years]) was calculated. The total amount of iron removed by phlebotomy (IR) was estimated as previously reported /25/. The geographical origin of the patients was ascertained up to the fourth generation.

In molecular studies genomic DNA was extracted from peripheral blood leukocytes. C282Y and H63D mutations were detected using standard polymerase chain reaction (PCR) and restriction enzyme digestion with Rsa I and Bcl I, respectively /18/. Microsatellites D6S265, D6S105 and D6S1281 were analysed as described /26/. HLA-A antigens were defined by the microlymphotoxicity test. Haplotypes were constructed manually on the basis of intrafamilial segregation of the marker alleles.

The entire coding sequence and exon-intron boundaries of the HFE gene were amplified by PCR according to Carella et al /18/. The nucleotide sequence of both DNA strands was independently determined in all the probands and in relatives with the C282Y negative chromosome identical to the proband by using the dideoxy chain-termination reaction with Sequenase version 2.0 (USB Amersham, Cleveland,Ohio) and 35 S-a-dATP, according to the protocol recommended by the manufacturer. Reactions were separated on a 6% denaturing polyacrylamide gel and visualised by autoradiography. The primers employed for sequencing were the same used for PCR reactions.

To further confirm the presence of the novel mutations (see results section) a restriction length polymorphism analysis (RFLP) was performed. Since the two mutations did not create or destroy a restriction endonuclease site, the portion of exon 3 containing the mutations was amplified by PCR using mismatched sense oligonucleotide specific for each mutation (Bria-F: 5' TGGCCCACCAAGCTGGACT 3'; Dom-F: 5' GCCTGGCCCACCAAACTG 3') to create an informative restriction site for BsrSI in the wild type HFE sequence. The antisense oligonucleotide was the same used for sequencing.The results are shown in the following table 2:

**Table 2. Data of the 5 probands affected by hemochromatosis.**

| | Sex | Age yrs | TS % | SF µg/L | Scheuer's grade | HIC µmol/g | HII | IR | Clinical complicat. |
|---|---|---|---|---|---|---|---|---|---|
| 1 | M | 48 | 96 | 694 | 4 | 238 | 4.9 | 4 | none |
| 2 | M | 47 | 79 | 1206 | 4 | 359 | 7.6 | 23 | C |
| 3 | M | 42 | 86 | 608 | 3 | 144 | 3.4 | 3.6 | none |
| 4 | M | 50 | 106 | 2740 | 4 | 425 | 8.5 | 21 | C,H,D |
| 5 | M | 66 | 100 | 1351 | 4 | 421 | 5.4 | - | C,H,A,D |

Legend. TS: transferrin saturation; SF: serum ferritin; HIC: hepatic iron concentration; HII**:** hepatic iron index; IR: iron removed; C: hepatic cirrhosis; H: hypogonadism; A: arthropathy; D: diabetes.

Table 2 reports iron and clinical data of the 5 probands. Three patients (1, 2 and 3) originated from the same subalpine valley in the north west (Ossola) and two (4 and 5) from an area around the city of Monza (Brianza). Sequence analyses of the HFE gene in probands showed two novel nonsense mutations in exon 3 in the heterozygous state. The first one, a G→ T mutation, at the nucleotide 502 of the HFE cDNA sequence (codon 168) (GAG→ TAG, Glu→ Stop) was found in probands from the Ossola valley. The second, a G→ A mutation at nucleotide 506 of HFE cDNA sequence (codon 169) (TGG→ TAG, Trip→ Stop) was detected in probands from Brianza region. The new variants were named HFE-Ossola and HFE-Brianza from the origin of the patients.

In one family an affected brother who shared the same chromosome 6p haplotypes and the same HFE mutations with the proband was found. His HIC was 176.2 µmol/g and the HII 3.92.

The two new variants were in complete linkage disequilibrium with the C282Y and H63D mutations. HFE-Ossola was associated with haplotype D6S265-3, HLA-A24, D6S105-5 and D6S1281-6 in two families, whereas in the third family, the haplotype changed at D6S265 and HLA-A loci suggesting that a recombination event occurred between HLA-A and D6S105. HFE-Brianza was associated with haplotype D6S265-3, HLA-A24, D6S105-6 and D6S1281-6.

The two mutations determine a stop codon at nucleotide 502 and 506 of the open reading frame (nt 502 GAG→ TAG and nt 506 TGG→ TAG) respectively, leading to proteins that lack the a3 domain, the transmembrane domain and the cytoplasmic tail. These mutations probably produce, differently from C282Y, a complete disruption of the function of HFE similar to that induced by the partial deletion of exon 4 in HFE-deficient mice /27,28/. Thus, these mutated alleles may behave as a null allele. It is expected that the combination of these mutations in the compound heterozygous state with C282Y leads to severe phenotype expression. Three of the five probands (2, 4 and 5) had indeed a severe disease characterized by the presence of high amount of iron overload and HH-related clinical complications. Proband 3 had a mild phenotype probably due to the fact he was a blood donor for the last 10 years, whereas proband 1 and his affected brother had a relatively mild phenotype and no other obvious protective factors such as blood loss or malabsorption.

Thus, phenotype variability exists in these patients and could be related to both environmental and genetic factors. The existence of modifying genes located around the D6S105 region which influence phenotypic expression of C282Y homozygous HH patients has been recently proposed /25,29/, but further studies are needed to clarify genotype-phenotype correlations in HH patients.

Heterozygotes for HFE-Ossola and HFE-Brianza mutations did not have elevated serum iron indices indicating that neither mutation was able to produce iron overload in the heterozygous state. Moreover, the three compound heterozygotes for either HFE-Ossola or HFE-Brianza and H63D did not show evidence of iron overload. Since they were females and/or young persons, it is possible that, as occur for C282Y, the combination of the two nonsense mutations with H63D or other mild HFE mutations may produce a HH phenotype with low penetrance /17/.

This is the first reported finding of nonsense mutations of the HFE gene. Moreover, this is the first description of several unrelated probands affected by HH who are compound heterozygotes for C282Y and a mutation other than H63D. The two mutations here reported probably originated in the two northern Italian regions and increased in frequency as a result of a local founder effect. Since no consanguinity is present up to the fourth generations between the affected families within each of the two geographical areas, the two mutations should be arisen much more than 100 years ago. Analysis of the frequency of HFE genotypes in HH probands originating from the two Italian regions of Ossola and Brianza shows that compound heterozygotes for C282Y and HFE-Ossola or HFE-Brianza are more frequent than or at least as frequent as C282Y/H63D compound heterozygotes (data not shown). In fact, C282Y/HFE-Ossola compound heterozygotes account for 25% of HH probands in the Ossola region (Vs 8.3% of C282Y/H63D), and C282Y/HFE-Brianza and C282Y/H63D compound heterozygotes each account for 8.4% of HH probands in Brianza region. Whereas the low frequency of C282Y/H63D compound heterozygotes is due to the very low penetrance of this genotype (from 0.44% to 1.5% in different populations) /17/ it is expected that C282Y/HFE-Ossola or HFE-Brianza compound heterozygotes have a 100% penetrance due to the severity of both mutations. These results confirm that HH in Italy is not as homogeneous as in northern Europe and underlines the possibility of other mutations of HFE that may have different relevance in different countries. The recognition of these mutations may have practical implications in diagnostic and screening strategies for HH.

### References

1. Feder J.N., Gnirke A., Thomas W., Tsuchihashi Z, Ruddy D.A., Basava A., Dormishian F., Domingo R., Ellis M.C., Fullan A., Hinton L.M., Jones N.L., Kimmel B.E., Kronmal G.S., Lauer P., Lee V.K., Loeb D.B., Mapa F.A., McClelland E., Meyer N.C., Mintier G.A., Moeller N., Moore T., MoriKang E., Prass C.E., Quintana L., Starnes S.M., Schatzman R.C., Brunke K.J., Drayana D.T., Risch N.J., Bacon B.R., Wolff R.K.. A novel MHC class I-like gene is mutated in patients with hereditary haemochromatosis. Nat Genet 1996;13:399-408.
2. Jazwinska, E.C., Cullen, L.M., Busfield, F., Pyper, W.R., Webb, S.I., Powell, L.W., Morris, C.P., and Walsh, T.P. (1997). Haemochromatosis and HLA-H. Nature Genet. 14, 249-251.
3. Obericanins C., Kazemi-Shirazi L., Kury F., Polli C., Maier-Dobersberger T., Yeganehfar C., and Ferenci P. (1997). Genotyping of the hereditary haemochromatosis mutation (HLA-H:C282Y) in microtiter plates. Am. J. Hum. Genet. 61, A225 (Suppl.).
4. Steffensen R., Varming K., and Jersild C. (1998). Determination of gene frequencies for two common haemochromatosis mutations in the Danish population by a novel polymerase chain reaction with sequence-specific primers. Tissue Antigens 52, 230-235.
5. Bollhalder M., Mura C., Landt O., and Maly F.E. (1999). LightCycler PCR assay for simultaneous detection of the H63D and S65C mutations in the HFE hemochromatosis gene based on opposite melting temperature. Clin. Chem. 45, 2275-2278.
6. Christiansen L., Bygum A., Thomsen K., Brandrup F., Horder M. and Petersen N.E. (1999). Denaturing gradient gel electrophoresis analysis of the hemochromatosis (HFE) gene: impact of HFE gene mutations on the manifestation of porphyria cutanea tarda. Clin. Chem. 45, 2025-2026.
7. Villiers J.N., Hillermann R., Loubser L., Kotze M.J. Spectrum of mutations in the HFE gene implicated in haemochromatosis and porphyria. Hum Mol Genet 1999;8:1517-22.
8. Mangasser-Stephan K., Tag C., Reiser A., and Gressner A.M. (1999). Rapid genotyping of haemochromatosis gene mutations on the LightCycler with fluorescent hybridization probes. Clin. Chem. 45,1875-1878.
9. Mura C., Raguenes O., Ferec C.. HFE mutations analysis in 711 hemochromatosis probands: evidence for S65C implication in mild form of hemochromatosis. Blood 1999;93:2502-5.
10. Miller S.A., Dykes D.D., and Polesky H.F. (1988). A simple salting out procedure for extracting DNA from human nucleated cells. Nucleic Acids Res. 16, 1215.
11. Bacon B.R., Powell L.W., Adams P.C., Kresina T.F., Hoofnagle J.H. Molecular medicine and hemochromatosis: at the crossroads. Gastroenterology 1999;116:193-207.
13. Jouanolle A.M., Gandon G., Jezeque P., Blayau M., Campion M.L., Yaouanq Y., Mosser J., Fergelot P., Chauvel B., Bouric P., Cam G., Andrieux N., Gicquel I., Le Gall J.Y., David V. Haemochromatosis and HLA-H. Nat Genet 1996;14:251-2.
14. Beutler E., Gelbert T., West C., Lee P., Adams M., Blackston R., Pockros P., Kosty M., Venditti C.P., Phatak P.D., Seese N.K., Chomey K.A., Ten Elshof A.E., Gerhard G.S., Chorney M. Mutation analysis in hereditary hemochromatosis. Blood Cells Mol Dis 1996;22:187-94.
15. Pietrangelo A., Camaschella C. Molecular genetics and control of iron metabolism in Hemochromatosis. Haematologica 1998;83:456-61.
16. Beutler E. Genetic iron beyond haemocromatosis: clinical effects of HLA-H mutations. Lancet 1997;349:296-7.
17. Beutler E. The significance of 187G (H63D) mutation in hemochromatosis. Am J Hum Genet 1997;61:762-4.
18. Carella M., D'Ambrosio L., Totaro A., Grifa A., Valentino M.A., Pipemo A., Girelli D., Roetto A., Franco B., Gasparini P., Camaschella C. Mutation analysis of HLA-H gene in Italian hemochromatosis patients. Am J Hum Genet 1997;60:828-32.
19. Pipemo A., Sampietro M., Pietrangelo A., Arosio C., Lupica L., Montosi G., Vergani A., Fraquelli M., Girelli D., Pasquero P., Roetto A., Gasparini P., Fargion S., Conte D., Camaschella C. Heterogeneity of hemochromatosis in Italy. Gastroenterology 1998;114:996-1002.
20. Camaschella C., Fargion S., Sampietro M., Roetto A., Bosio S., Garozzo G., Arosio C., Pipemo A. Inherited HFE-unrelated hemochromatosis in Italian families. Hepatology 1999;29:1563-4.
21. Pietrangelo A., Montosi G., Totaro A., Garuti C., Conte D., Cassanelli S., Fraquelli M., Sardini C., Vasta F., Gasparini P. Hereditary Hemochromatosis in adults without pathogenic mutations in the hemochromatosis gene. New Engl J Med 1999;341:725-32.
22. Wallace D.F., Dooley J.S,. Walker A.P. A novel mutation of HFE explains the classical phenotype of genetic hemochromatosis in a C282Y heterozygote. Gastroenterology 1999;116:1409-12.
23. Barton J.C., Sawada-Hirai R., Rothenberg B.E., Acton R.T. Two novel missense mutations of the HFE gene (I105T and G93R) and identification of the S65C mutation in Alabama hemochromatosis probands. Blood Cells Mol Dis 1999;25:146-54.
24. Scheuer P.J., Williams R., Muir A.R. Hepatic pathology in relatives of patients with hemochromatosis. J. Pathol Bacteriol 1962;84:53-64.
25. Pipemo A., Arosio C., Fargion S., Roetto A., Nicoli C., Girelli D., Sbaiz L., Gasparini P., Boari G., Sampietro M., Camaschella C.. The ancestral hemochromatosis haplotype is associated with a severe phenotype expression in italian patients. Hepatology 1996;24:43-6.
26. Camaschella C., Roetto A., Ciciliano M., Pasquero P., Bosio S., Gubetta L., Di Vito F., Girelli D., Totaro A., Carella M., Grifa A., Gasparini P. Juvenile and adult hemochromatosis are distinct genetic disorders. Eur J Hum Genet 1997;5:371-5.
27. Zhou X.Y., Tomatsu S., Fleming R.E., Parkkila S., Waheed A., Jiang J., Fei Y., Brunt E.M., Ruddy D.A., Prass C.E., Schatzman R.C., O'Neill R., Britton R.S,. Bacon B.R., Sly W.S. HFE gene Knockout produces mouse model of hereditary hemochromatosis. Proc Natl Acad Sci USA 1998;95:2492-7.
28. Levy J.E., Montross L.K., Cohen D.E., Fleming M.D., Andrews N.C.. The C282Y mutation causing hereditary hemochromatosis does not produce a null allele. Blood 1999;94:9-11.
29. Pratiwi R., Fletcher L.M., Pyper W.R., Do K.A, .Crawford D., Powell L.W., Jazwinska E.C. Linkage disequilibrium analysis in Australian haemochromatosis patients indicates bipartite association with clinical expression. Journal of Hepatology 1999;31:39-46.
30. Chehab F.F., and Wall J. (1992). Detection of multiple cystic fibrosis mutations by reverse dot blot hybridization: a technology for carrier screening. Hum. Genet. 89, 163-168.
31. Douabin V., Moirand R., Jouanolle A.M., Brissot P., Le Gall J.Y., Deugnier Y., and David V. (1999). Polymorphisms in the HFE gene. Hum. Hered. 49, 21-26.
32. Jeffrey G.P., Chakrabarti S., Hegele R.A., and Adams P.C. (1999). Polymorphism in intron 4 of HFE may cause overestimation of C282Y homozygote prevalence in haemochromatosis. Nature Genet. 22, 325-326.
33. Maggio A., Giambona A., Cai S.P., Wall J., Kan Y.W., and Chehab F.F. (1993). Rapid and simultaneous typing of hemoglobin S, hemoglobin C, and seven Mediterranean (-thalassemia mutations by covalent reverse dot-blot analysis: application to prenatal diagnosis in Sicily. Blood 81, 239-242.
34. Saiki R.K., Walsh P.S., Levenson C.H., and Erlich H.A. (1989). Genetic analysis of amplified DNA with immobilized sequence-specific oligonucleotide probes. Proc. Natl. Acad. Sci. USA 86, 6230-6234.
35. Stuyver L., Rossau R., Wyseur A., Duhamel M., Vanderborght B., Heuverswyn H.V., and Maertens G. (1993). Typing of hepatitis C virus isolates and characterization of new subtypes using a line probe assay. J. Gen. Virol.
36. Lebron J.A., Bennett M.J., Vaughn D.E. et al Crystal structure of the hemochromatosis protein HFE and characterization of its interaction with transferrin receptor. Cell. 1998, 93:111-123.
41. Rubini, M. Carturan S., Barp L., Bononi I., Baricordi O., Europ.J.Hum.Genet. (2000), Vol.8 (Suppl. 1), p.158.

## Claims

1. Probe for the diagnosis of hemochromatosis **characterized by** the probe being capable of hybridizing with nucleic acids of a biological sample in a region corresponding to a region of the HFE cDNA sequence containing nucleotide 502 to determine if a G→ T mutation exists at position 502 of the HFE cDNA sequence.

## Patentansprüche

1. Sonde zur Diagnose von Hämochromatose, **dadurch gekennzeichnet, dass** die Probe mit Nukleinsäuren einer biologischen Probe in einer Region, die der Region der HFE cDNA Sequenz enthaltend Nuleotid 502 entspricht, hybridisieren kann, um zu bestimmen, ob eine G→T Mutation bei Position 502 der HFE cDNA Sequenz existiert.

## Revendications

1. Sonde destinée au diagnostic de l'hémochromatose, **caractérisée par le fait que** la sonde est capable de s'hybrider aux acides nucléiques d'un échantillon biologique dans une région correspondant à une région de la séquence d'ADNc du gène HFE contenant le nucléotide 502 pour déterminer si une mutation G→ T existe au niveau de la position 502 de la séquence d'ADNc du gène HFE.
